# EUROPEAN PATENT SPECIFICATION

(11) **EP 0 760 671 B1**
(45) Date of publication and mention of the grant of the patent: **17.08.2005**
(21) Application number: 95920641.8
(22) Date of filing: 24.05.1995
(51) Int. Cl.: A61K 38/00, A61K 38/04, A61K 39/00, A61K 39/108, A61K 39/385, C07K 9/00, C07K 14/245, C07K 17/00, C07K 19/00

(54) **PEPTIDES USED AS CARRIERS IN IMMUNOGENIC CONSTRUCTS SUITABLE FOR DEVELOPMENT OF SYNTHETIC VACCINES**
PEPTIDE, VERWENDET ALS TRÄGER IN IMMUNOGENEN KONSTRUKTEN, DIE IN DER ENTWICKLUNG SYNTHETISCHER IMPFSTOFFE GEEIGNET SIND
PEPTIDES UTILISES COMME PORTEURS DANS DES PRODUITS DE SYNTHESE IMMUNOGENES POUR LE DEVELOPPEMENT DE VACCINS DE SYNTHESE

(30) Priority: 25.05.1994 IL 10979094
(43) Date of publication of application: 12.03.1997
(73) Proprietor: YEDA RESEARCH & DEVELOPMENT COMPANY, LTD., 76100 Rehovot (IL)
(72) Inventor: COHEN, Irun R., 76354 Rehovot (IL); FRIDKIN, Matityahu, 76284 Rehovot (IL); KONEN-WAISMAN, Stephanie, 65214 Tel Aviv (IL)
(74) Representative: Horner, Martin Grenville
(86) International application number: PCT/US1995/006575
(87) International publication number: WO 1995/031994

(56) References cited:
- WO-A-93/17712
- WO-A-94/03208
- LUSSOW A.R. ET AL: "Mycobacterial heat-shock proteins as carrier molecules" EUROPEAN JOURNAL OF IMMUNOLOGY, vol. 21, 1991, pages 2297-2302, XP002114157
- STEPHANIE KNEN-WAISMAN ET AL: "SELF AND FOREIGN 60-KILODALTON HEAT SHOCK PROTEIN T CELL EPITOPE PEPTIDES SERVE AS IMMUNOGENIC CARRIERS FOR A T CELL-INDEPENDENT SUGAR ANTIGEN" JOURNAL OF IMMUNOLOGY, vol. 154, no. 11, 1 January 1995 (1995-01-01), pages 5977-5985, XP002083262 ISSN: 0022-1767
- HEMMINGSEN S.M. ET AL: "Homologous plant and bacterial proteins chaperone oligomeric protein assembly" NATURE, vol. 333, 1988, pages 330-334, XP002114158
- IMMUNOLOGY LETTERS, Vol. 25, issued 1990, A.R. LUSSOW et al., "Towards Vaccine Optimization", pages 255-264.
- PROC. NATL. ACAD. SCI. U.S.A., Vol. 85, issued June 1988, D. YOUNG et al., "Stress Proteins are Immune Targets in Leprosy and Tuberculosis", pages 4267-70.

## Description

### FIELD OF THE INVENTION

A synthetic peptide, is described having an amino acid sequence corresponding to that of a T cell epitope of the heat shock protein 65 of *E. coli* (hereinafter GroEL) and its analogs able to be recognized in association with a range of mouse major histocompatibility complex (MHC) molecules. Said peptide or its analogs can be used as synthetic carriers in the preparation of immunogenic conjugates consisting of said peptides and a natural or synthetic hapten derived from a pathogenic agent of interest.

### BACKGROUND OF THE INVENTION

Immunization against infection caused by pathogenic microorganism (bacteria, viruses and parasites) is generally achieved by inoculating an individual with the natural antigen (attenuated or killed microorganism) or parts of said infectious agent (for example detoxified microbial products) in order to stimulate a protective immune response able to neutralize the pathogenic microbe or its deleterious effects.

Limited availability of the natural antigenic substance, risks involved in handling pathogenic material as well as storage problems stimulated the interest in the development of subunit vaccines. Isolated protective epitopes nevertheless are often characterized by their poor immunogenicity. The carbohydrate capsules of bacteria are an example of such coats: They are not easily recognized by T cells and therefore the immune response to these antigens is deprived of T cell help, T cell memory, IgG class switch, and affinity maturation. Such an immune response is inefficient and resistance to infection with bacteria encoated with carbohydrate capsules is not easily obtained by vaccination with bacterial carbohydrates. Peptide epitopes too may be poorly immunogenic, the absence of a T cell epitope and the genetically restricted immune response being the reason.

It is now well established that most antigens require T cell help to induce B cells to produce antibodies. Conjugating a "helper" or T cell determinant to a B cell-specific antigen was shown to induce humoral immune responses to the coupled B cell epitope. The discovery by Avery & Goebel (1929) that coupling of polysaccharides to protein carriers increases immunogenicity has recently been used for the preparation of vaccines for human use. Both in humans and in rodents these conjugates behave like T cell dependent antigens by exhibition of immunological memory. There are similarities between conjugate polysaccharide vaccines and protein carrier-hapten systems. Thus the capsular polysaccharide (CPS) conjugates are able to induce protective levels of CPS antibodies in infants, while CPS alone is not. It is possible that the superior immunogenicity of conjugates compared to that of pure polysaccharides is due to the help by carrier-specific T cells, as has been demonstrated in the carrier-hapten system in rodents.

In most cases, T cell independent (T-ind) antigens have been coupled to large immunogenic carrier proteins such as tetanus toxoid, cholera toxin or diphtheria toxoid. Nevertheless, besides dosage limitations and the risk of sensitization to the carrier itself, as reported for tetanus toxoid, the immunological response to high molecular weight carrier molecules harboring stimulatory as well as suppressive T cell epitopes is not very predictable. It has been shown that the antibody response to a hapten coupled to a carrier protein can also be inhibited when the recipient has been previously immunized with the unmodified protein. This phenomenon has been termed carrier-induced epitope suppression and was recently demonstrated to occur with a number of hapten-protein conjugates (Herzenberg & Tokuhisa, 1982). Since the development of more potent conjugate vaccines against a large number of extremely infectious organisms is still important, efforts are being made to search for more appropriate carrier molecules providing the needed T cell epitopes. Universally immunogenic T cell epitopes, defined by specific peptides with sharply outlined immunological characteristics, might represent a new generation of such alternative molecules. T cell epitopes of various sorts have been used for this purpose before. However, to trigger a strong memory response when the host meets the infectious agent after vaccination, the T cell carrier epitope should be present along with the specific B cell epitope. This fact would seem to require that a different T cell carrier be used for each infectious agent. Highly abundant proteins well recognized by the immune system might be an appropriate source for peptides serving this purpose.

Studies using a wide variety of proteins, both those closely related to self and those phylogenetically distantly related, have shown that the majority of T cells are focused onto a few immunodominant epitopes with a minority responding to other, subdominant determinants. This hierarchy of determinant utilization by T cells could result from a combination of factors including differential affinities for the available MHC molecules, the diversity of the T cell repertoire, internal competition for MHC-binding sites and fine differences in processing (Babitt et al, 1985; Kappler et al, 1987; Brett et al, 1988)

Evidence is accumulating that proteins belonging to the family of heat shock proteins (hsp's) are major antigens of many pathogens (Young et al, 1988). Hsp's were first described and later named due to their production by cells exposed to sudden elevations in temperature. The hsp's include proteins of various molecular weights, including 20kD, 60kD, 65-68kD, 70kD, 90kD, 110kD, and others. It is now apparent that hsp's are induced in all cells by many different environmental insults, including oxidative injury, nutrient depletion and infection with intracellular pathogens; the hsp response enables the cell to survive under otherwise unfavorable conditions. Although cellular stress increases the synthesis of hsp's, many hsp's are also constitutively expressed and play an essential role in normal cell function. The hsp response is ubiquitous throughout the pro- and eukaryotic kingdoms and hsp's belong to some of the most conserved molecules.

Hsp65, as a representative member of the proteins belonging to the hsp family, can be considered to be a dominant antigen because infection or immunization with many different bacteria induces antibodies and T cells specific for the hsp65 molecule (Young et al, 1988). In mice immunized with Mycobacterium tuberculosis, 20% of all T cells which respond to the bacterium, are specific for hsp65. Interestingly, T cells with reactivity to hsp65 have also been identified in normal healthy individuals lacking any clinical signs of disease (Munk et al, 1988).

Lussow et al. (1990) showed that priming of mice with live Mycobacterium tuberculosis var.bovis (BCG) and immunization with the repetitive malaria synthetic peptide (NANP)₄₀, conjugated to purified protein derivative (PPD), led to the induction of high and long-lasting titers of anti-peptide IgG antibodies. Later on, Lussow et al. (1991) reported that the mycobacterial hsp65 as well as the hsp65 of *E. coli* (GroEL) acted as carrier molecules in mice, previously primed with BCG, for the induction of high and long-lasting titers of IgG against the repetitive malaria synthetic peptide (NANP)₄₀. Anti-peptide antibodies were induced when the malaria peptide, conjugated to the mycobacterial or *E. coli* hsp, was given in the absence of any adjuvants.

Barrios et al. (1992) have shown that mice immunized with peptides or oligosaccharides conjugated to the 70kD hsp produced high titers of IgG antibodies in the absence of any previous priming with BCG. The anti-peptide antibody response persisted for at least 1 year. This adjuvant-free carrier effect of the 70kD hsp was T cell dependent, since no anti-peptide nor anti70kD IgG antibodies were induced in athymic nu/nu mice. Previous immunization of mice with the 65kD or 70kD hsp did not have any negative effect on the induction of anti-peptide IgG antibodies after immunization with hsp-peptide conjugates in the absence of adjuvants. Furthermore, preimmunization with the 65kD hsp could substitute for BCG in providing an effective priming for the induction of ariti-(NANP)₄₀ antibodies. The carrier effect of mycobacterial hsp65 and hsp70 for conjugated peptides was demonstrated also in non-human primates (Perraut et al, 1993).

It can be assumed that some T cell epitopes within the sequence of the bacterial hsp65 protein show immunodominance and are able to induce immunological memory, whereas others do not express privileged immunological recognition or are involved in the induction of autoimmune diseases. Distinguishing between functionally different T cell epitopes, binding to several different MHC molecules, may lead to the identification of universally immunogenic peptides, which can qualify as safe, defined, and potent alternatives for carrier molecules of T-ind antigens.

Israel Patent Application No. 102687 of the same applicants describes specific T cell epitopes of human hsp65, and analogs thereof, conjugated to poorly immunogenic molecules.

None of the above mentioned references describes specific T cell epitopes derived from the sequence of hsp65 of *E. coli* (GroEL) conjugated to poorly immunogenic molecules.

### SUMMARY OF THE INVENTION

It is an object of the present invention to provide a method for enhancing the immunogenicity of poorly immunogenic antigen molecules, thus converting them to suitable antigens for immunization.

For this purpose, the present invention provides conjugates of a poorly immunogenic antigen and a synthetic peptide carrier constituting a T cell epitope derived from the sequence of E. *coli* hsp65 (GroEL) Pep278e or an analog thereof, wherein at least 70% of the electric properties and of the hydrophobicity of Pep278e are conserved, said peptide or analog being capable of increasing substantially the immunogenicity of the poorly immunogenic antigen.

Any peptide, or analog thereof, derived from GroEL Pep278e peptide constituting a T cell epitope and able to increase substantially the immunogenicity of the poorly immunogenic antigen, can be used in the invention.

The peptide according to the invention, herein designated 278e, corresponds to positions 437-453 of the GroEL molecule, and has the sequence:

437                                    453
N E D Q N V G I K V A L R A M E A

The poorly immunogenic antigen molecule may be a peptide, a polypeptide or a protein, e.g., a peptide derived from HIV virus or from malaria antigen, or a bacterial polysaccharide, e.g., capsular polysaccharides from Haemophilus influenzas type b, Streptococcus pneumoniae, Neisseria meningitides, group B Streptococci, *E. coli* type K1, Salmonella, such as Salmonella typhi, etc.

The carrier peptide is covalently linked to the poorly immunogenic antigen molecule, either directly or through a spacer.

The invention further relates to vaccines comprising a conjugate of the invention or a mixture of the poorly immunogenic antigen and the suitable peptide carrier.

In another embodiment, the invention relates to a method of immunization of a mammalian host which comprises administering to said host an effective amount of a conjugate of the invention, or co-administering effective amounts of a poorly immunogenic antigen molecule and of a synthetic peptide carrier constituting a T cell epitope derived from the sequence of GroEL Pep278e peptide, or an analog thereof, said peptide or analog being able to enhance substantially the immunogenicity of the poorly immunogenic antigen.

### BRIEF DESCRIPTION OF THE DRAWINGS

Fig. 1 shows lymph node proliferation to 278 epitopes 278e, 278m and 278cox after immunizing BALB/C mice with 20 µg 278 epitope emulsified in incomplete Freund's adjuvant (IFA).
Figs. 2a-c show lymph node proliferation to 278e and to control peptide ACR 259-271 after immunizing B10RIII mice (2a), B10.BR mice (2b), and B10.S mice (2c) with 20 µg 278e emulsified in IFA.
Fig. 3 illustrates lymph node proliferation to peptides 278e, 278m, and AcR259-271 after immunizing BALB/c mice with 2µg Vi-fragments conjugated to 278 homologous or with 2µg Vi-fragments alone.
Fig. 4 illustrates lymph node proliferation to peptides 278e, 278m, and AcR259-271 after immunizing BALB/c mice with 20 µg Vi-fragments conjugated to 278 homologous or with 2µgVi-fragments alone.
Fig. 5 shows the serum anti-Vi IgG antibody response induced in BALB/c mice by Vi-fragments alone or Vi-fragment-conjugates 278h-Vi, 278m-Vi, and 278e-Vi. The injected polysaccharide amount in each group was 2µg.
Primary and secondary immune responses are depicted. Results are shown at a serum dilution 1:100.

### DETAILED DESCRIPTION OF THE INVENTION

Preferred conjugates according to the invention are formed by covalently linking peptide 278e with a bacterial polysaccharide, e.g., the capsular polysaccharide (CPS) Vi of Salmonella typhi, hereinafter referred to as Vi or Vi-fragments, a linear homopolymer of poly-α-(1-4)GalNAc variably O-acetylated at the C₃-position, as shown in scheme 1. The native Vi molecule has a molecular weight of about 3 x 10³ kD (Vi). Vi-fragments (about 45kD) are prepared by ultrasonic cleavage, which does not alter the structure of its monomeric units and which produces a relatively homogeneous polysaccharide (Stone & Szu, 1988). Vi/Vi-fragments alone, like other CPSs, do not elicit a booster response in mammals, either in animals or in humans, when reinjected, but its immunogenicity is increased when presented as a conjugate according to the invention coupled to a suitable peptide derived from GroEL or an analog thereof, or in a mixture with such a peptide or analog. Reinjection of the Vi-peptide conjugate induces an increase in the level of anti-Vi antibodies (booster effect), which are mainly represented by the IgG isotype.

Peptide 278e of the present invention is clearly distinct from peptides 278h and 278m of above-mentioned Israel Patent Application No. 102687.

278e N E D Q N V G I K V A L R A M E A
278h N E D Q K I G I E I I K R T L K I
278m N E D Q K I G I E I I K R A L K I

Peptide 278e is a highly charged and hydrophobic molecule. Thus, 5 out of 17 constituent amino acids are ionized (3 negatively and 2 positively) at physiological pH. Five amino acid residues are hydrophobic. In addition, 3 residues are amidated and capable of establishing substantial hydrogen bonding. The peptide is further characterized as possessing a polar negatively-charged N-terminal domain, a polar charged C-terminal domain and a highly hydrophobic core. 278e can be modified while retaining activity. In order to preserve activity, however, its overall structural features should be maintained. Thus, positions 2, 3 and 16 can be either occupied by either E or D, and positions 9 and 13 by either K or R. Conservation of the charge at positions 9 and 13 (positive to negative and vice-versa) will lead to active peptides. A hydrogen bond forming amino acid, preferably N and Q, should occupy positions 1 and 4.

Hydrophobicity at positions 6, 8, 10, 12 and 15 should be maintained by incorporating hydrophobic amino acids, natural, e.g., I, L, V, M or F, or unnatural, *e.g.,* norleucine (Nle) or norvaline (Nva).

The term "analogs" in the present invention relates to Pep278e peptides obtained by replacement, deletion or addition of amino acid residues to the sequence of the T cell epitope, as long as they have the capability of enhancing substantially the immunogenicity of poorly immunogenic antigen molecules. Analogs, in the case of peptide 278e, are peptides such that at least 70%, preferably 90-100%, of the electric properties and of the hydrophobicity of the peptide molecule are conserved. These peptides can be obtained according to the instructions in the paragraph herein before.

The peptides according to the invention may have all the optically active amino acid residues in L or D form, or some of the amino acid residues are in L and others are in D form.

By "substantially increasing the immunogenicity of a poorly immunogenic antigen molecule" it is meant to comprise both the induction of an increase in the level of antibodies against said antigen as well as the presentation of said antibodies as mainly of the IgG isotype.

The peptide carrier may be linked to the antigen molecule directly or through a spacer.

A direct link between the peptide and Vi or Vi-fragments is shown in Scheme 1 herein, where the conjugate 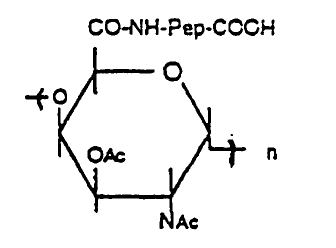 is obtained by Procedure 1 as described hereafter.

The spacer may have the formula -O-R-CO or -NH-R-CO, thus forming an ester or amide, respectively, with the carboxyl group of Vi or Vi-fragments and a peptide bond with the terminal amino group of the peptide; or -NH-R-CH₂-, wherein R is a saturated or unsaturated hydrocarbon chain optionally substituted and/or interrupted by one or more aromatic radicals or by heteroatoms such as O, S or N. Preferably, R is an aliphatic hydrocarbon chain containing 3-16 carbon atoms, such as the residue of ε-aminocaproic acid.

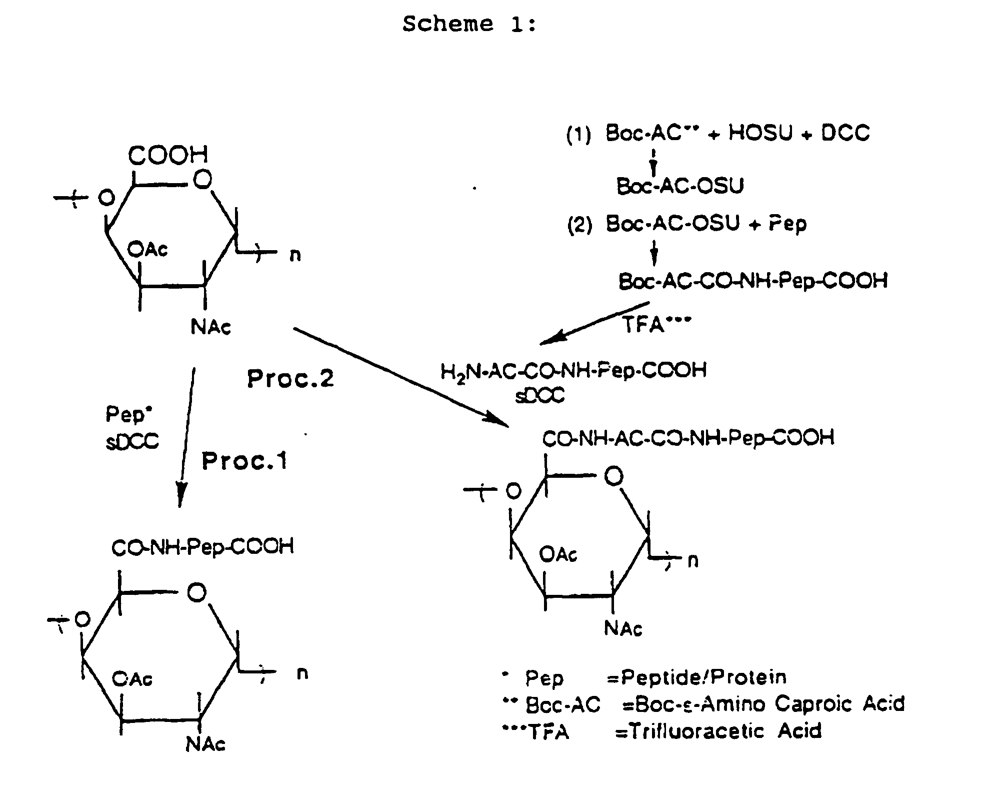

The conjugate of the formula: 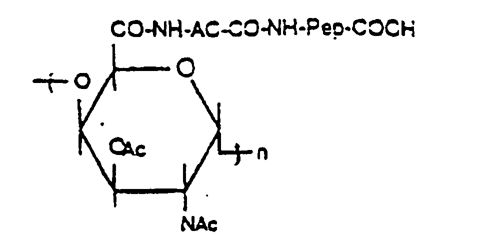 in which Ac is acetyl, AC is the residue of ε-aminocaproic acid, Pep is the residue of the peptide carrier 278e or an analog thereof and the saccharide residue represents a repeating unit of the Vi capsular polysaccharide (Vi or vi-fragments) of Salmonella typhi, may be prepared by Procedure 2 depicted in Scheme 1 and described in detail hereafter.

The conjugates wherein the spacer is -NH-R-CH₂- are obtained by reduction of -NH-R-CO- groups or by alkylation cm the peptidic amino terminus with -NH-R-CH₂-X, when X is an appropriate leaving group such as an halide.

The invention further relates to vaccines comprising a conjugate of the invention. These vaccines may be administered by any suitable route, e.g., orally or via the subcutaneous route in suitable vehicles for human and veterinary purposes.

The invention will now be illustrated by the following non-limiting examples:

### EXAMPLES

In the examples, the following materials and methods will be used.

### Materials & Methods

**a.** **Materials:** All solvents and chemicals were of analytical grade and obtained from Aldrich, U.S.A., unless otherwise mentioned.
**b.** **Peptide synthesis****:** Peptide 278e was prepared with an automated synthesizer (Applied Biosystem model 430A, Germany) using the company's protocols for t-butyloxycarbonyl (BOC) strategy (Kent et al, 1984).

The following control peptides were synthesized:
Peptide 278h corresponding to positions 458-474 of the human hsp65 molecule, 278m corresponding to positions 458-474 of the murine hsp65, and 278cox corresponding to positions 437-453 of the Coxiella burnetti hsp65 protein, said control peptides having the sequences depicted below: 
278h N E D Q K I G I E I I K R T L K I
278m N E D Q K I G I E I I K R A L K I
278cox N E D Q R V G V E I A R R A M A Y
   A further control peptide, AcR259-271, corresponds to positions 259-271 of the murine acetylcholine receptor α-chain and has the sequence: V I V E L I P S T S S A V
   This peptide is recognized by T cells in the context of MHC class II molecules of the H-2d haplotype.
**c.** **Reversed-phase HPLC****:** The purity of the peptide products was estimated by using the analytical HPLC column RP18 (Merck, Darmstadt, Germany) employing the SP8750 liquid chromatography system equipped with a SP8733 variable wavelength detector in water-acetonitrile gradients containing 0.1% trifluoroacetic acid (TFA). The effluents were monitored by UV absorbance at 220 nm. Acetonitrile of HPLC grade was purchased from Merck (Darmstadt, Germany). Peptides were further analyzed by amino acid analysis.
**d.** **Vi:** The Vi purified from Citrobacter freundii WR7011 (kindly donated by J.B. Robbins and S.C. Szu, National Institute of Health, Bethesda, Maryland, U.S.A.) contained < 1% (each) protein, nucleic acid, and lipopolysaccharide. The molecular size of the Vi was estimated to be 3 X 10³ kD. The Vi-fragments of about 45kD were prepared by ultrasonic irradiation and were kindly provided by Dominique Schulz (Pasteur-Merieux, France).
**e.** **Coupling of Vi and Vi-fragments with peptide****:**
   Procedure 1 (see scheme 1) Conjugation of Vi/Vi-fragments and peptide without a spacer. One part of Vi/Vi-fragment and one part of peptide were dissolved in a minimal volume of double distilled water (ddw) and incubated for 12 hours at room temperature (RT) at pH 6 in the presence of two parts water-soluble carbodiimide (CDI; 1-(3-dimethylaminopropyl)-3-ethylcarbodiimide hydrochloride).
   After dialysis of the reaction mixture, the peptide density in the conjugate was determined by amino acid analysis and the sugar content of the construct estimated by Fourier transformed infrared spectroscopy (FTIR).
   Procedure 2 (see scheme 1) Coupling of Vi/Vi-fragments and peptide following extension of peptide chain by a spacer in solution. In order to activate the carboxyl-function of the tBoc-ε-aminocaproic acid (t-Boc-AC) by N-hydroxysuccinimide, 1 mmol t-Boc-AC was mixed with 1.15 mmol N-hydroxysuccinimide in a minimal volume of dioxane (Merck, Germany); 1.15 mmol N,N'-dicyclohexylcarbodiimide (DCC) dissolved in dioxane was added, and after 3 hours the reaction mixture was filtered and washed with dioxane. 0.1 mmol of the desired peptide was dissolved in a small amount of ddw and mixed with 0.2 mmol KHCO₃, (Merck). The solution, in dioxane, of the N-hydroxysuccinimide ester of t-BocAC and the prepared peptide solution were mixed and reacted for 1 hour with vigorous mixing. The reaction mixture was then diluted with ddw (10 ml), cooled and acidified with 1N KHSO₄, solution. The product was extracted by ethyl acetate. The organic solution was washed with ddw, dried over Na₂SO₄, and evaporated to dryness. After drying the product for 2 hours over P₂O₅, dissolving it with 4-5 ml TFA and reacting for 10 minutes, the liquid was evaporated in vacuum at 30°C. The compound was washed twice with CH₂Cl₂ and the fluid evaporated before drying 2-3 hours over P₂O₅. Subsequently, the peptide-AC product was dissolved in ddw and the pH adjusted to 8. Five mg N-hydroxysuccinimide ester (prepared as described in Procedure 2 of Israel Patent Application No.102687) of Vi/Vi-fragments were added. After several hours of incubation, the resulting Vi-AC-Peptide conjugate was dialysed against ddw. The peptide density in the conjugate was estimated by amino acid analysis.
**f.** **Immunization****:** Female mice belonging to different strains, 2-3 months old, were immunized subcutaneously (sc), two times 4 weeks apart with Vi/Vi-fragment alone or the Vi/Vi-fragment-conjugate. The injected amount of antigen varied from experiment to experiment and is indicated in the figures. The used adjuvant was in all cases IFA. Mice from each experimental group were bled 12 days after each injection.
**g.** **Serology****:** Vi/Vi-fragment antibody levels elicited in mice with native or conjugated Vi, were determined by an enzyme-linked immunosorbent assay (ELISA). Since negatively-charged polysaccharides do not attach well to the polystyrene commonly used in the solid-phase ELISA, positively charged methylated bovine serum albumin (BSA) was used to coat Vi/Vi-fragments on the solid surface with very little non-specific binding. In detail, 0.5 mg Vi were dissolved in 1 ml PBS and stirred for 1 hour at room temperature. Ten mg methylated BSA (Sigma) were suspended in 1 ml H₂O and the obtained solution filtered on a 0.8 µm filter. To prepare the coating solution, 1 ml of dissolved polysaccharide was stirred for 20 minutes at room temperature with 50 µl of the methylated BSA solution and subsequently diluted 1:20 in PBS. Nunclon delta Si microwell plates were coated for 3 hours at 37°C with 100 µl coating solution per well (2.5 µg Vi/well). The plates wee washed five times with PBS containing 0.33% Brij35 (Sigma) and blocked with a solution of PBS and 1% dried skimmed milk for 2 hours at 37°C. After washing, 100 µl aliquots of diluted unknown sera and of diluted standard serum (dilution buffer containing 1% skimmed milk and 0.33% Brij35 in PBS) were added and the plates were incubated for 1 hour at 37°C. Reference and test sera were applied to the plates in duplicate. The non-bound antibodies were removed by washing and a 1:5000 dilution of goat anti-mouse IgG Fab₂-alkaline phosphatase conjugate (Sigma), in the case of the test sera, and rabbit anti-horse IgG Fab₂ enzyme conjugate, in the case of the standard serum, was added to the plates (100µl/well). After an incubation of 2 hours at 37°C, the plates were washed and the bound antibody visualized by the addition of 100 µl substrate solution containing 0.6 mg/ml of p-nitrophenylphosphate (Sigma) in diethanolamine-H₂O pH 9.8. The enzyme reaction was stopped 20 minutes later by the addition of 10 µl 5N NaOH per well. Optical densities were read at 405 nm. The anti-Vi standard serum Burro 260, containing 550mg Vi antibody/ml, was prepared by multiple intravenous injections of formalin-fixed Salmonella typhi Ty2 (kindly donated by J.B. Robbins and S.C. Szu, NIH, Maryland, U.S.A.). The results obtained are expressed as optical density read at 405 nm.
**h.** **Lymph node Proliferation after peptide-immunization****:**
   Groups of 3 mice of the designated mouse strain were immunized sc into the footpads with 20 µg peptide emulsified in 0.2 ml IFA/PBS (0.1 ml/foot). Draining lymph nodes were taken 10 days later. Lymph node cells (LNC) of immunized mice 5 X 10⁶/well) were cultured in the presence of different antigens. Cultures were set up in 200 µl Eagles medium supplemented with 2 mM glutamine, nonessential amino acids, 1mM sodium pyruvate, 100 U/ml penicillin, 100 mg/ml streptomycin, 5 x 10⁵M β-mercaptoethanol (Fluka AG, Buchs, Switzerland) containing 1% of syngeneic normal mouse serum, in round bottom microtiter plates (Falcon). After four-five days incubation, ³H-thymidine (0.5 mCi of 5 Ci/mmol, Nuclear Research Center, Negev, Israel) was added. Sixteen hours later, cells were harvested and radioactivity was counted. Results are expressed as counts per minute (cpm) or as stimulation indices (SI). The SI was defined as the ratio of the mean cpm of test cultures (with antigen) and the mean cpm of control cultures (without antigen).

### EXAMPLE 1. Preparation of Vi-peptide conjugates

Conjugates of Vi/Vi-fragments with peptide 278e and the control peptides were prepared as described above.

The composition of the Vi-peptide conjugates is summarized in Table 1. The results presented in Table 1 indicate that the molar ratio of peptide per sugar monomer was variable. Peptide doses of 0.8-2.2 µg injected per mouse as sugar-peptide conjugate were shown to be most effective.

### EXAMPLE 2. Lymph node cell proliferation to peptide 278e in different mouse strains with varying major histocompatibility complex MHC) background.

2.1. Lymph node proliferation after immunization with free carrier peptide. In order to test if peptide 278e can be recognized by the immune system in the context of different alleles of the murine MHC, 2-3 month old female mice (three animals per group) were injected sc with 20 µg of free peptide 278e emulsified in IFA as described in Material & Methods herein and specific proliferation of lymph node cells to peptide 278e and control peptides.

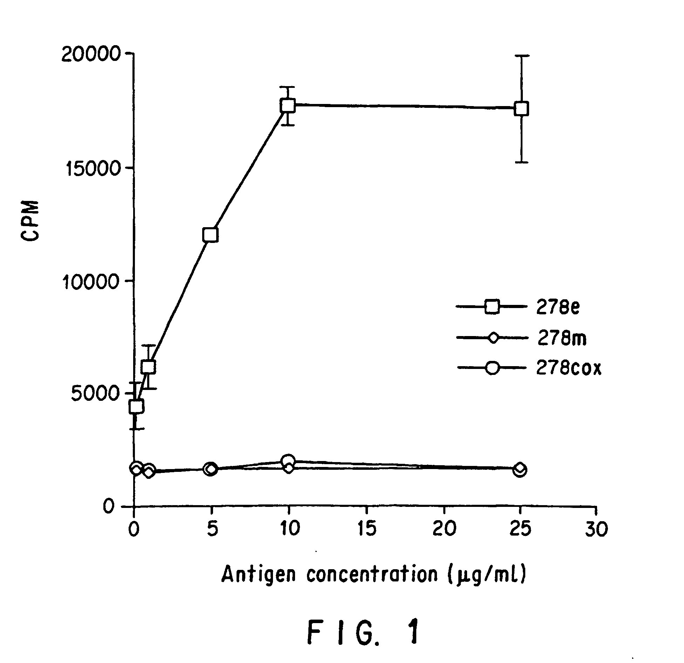

As shown in Fig. 1, LNCs of BALB/c (H-2d) mice inoculated with peptide 278e showed clear specific proliferative responses to the latter whereas no proliferation occurred to control peptide 278m and 278cox. Thus, LNCs primed with peptide 278e do not cross-react with the homologous self-peptide 278m derived from the sequence of murine hsp65.

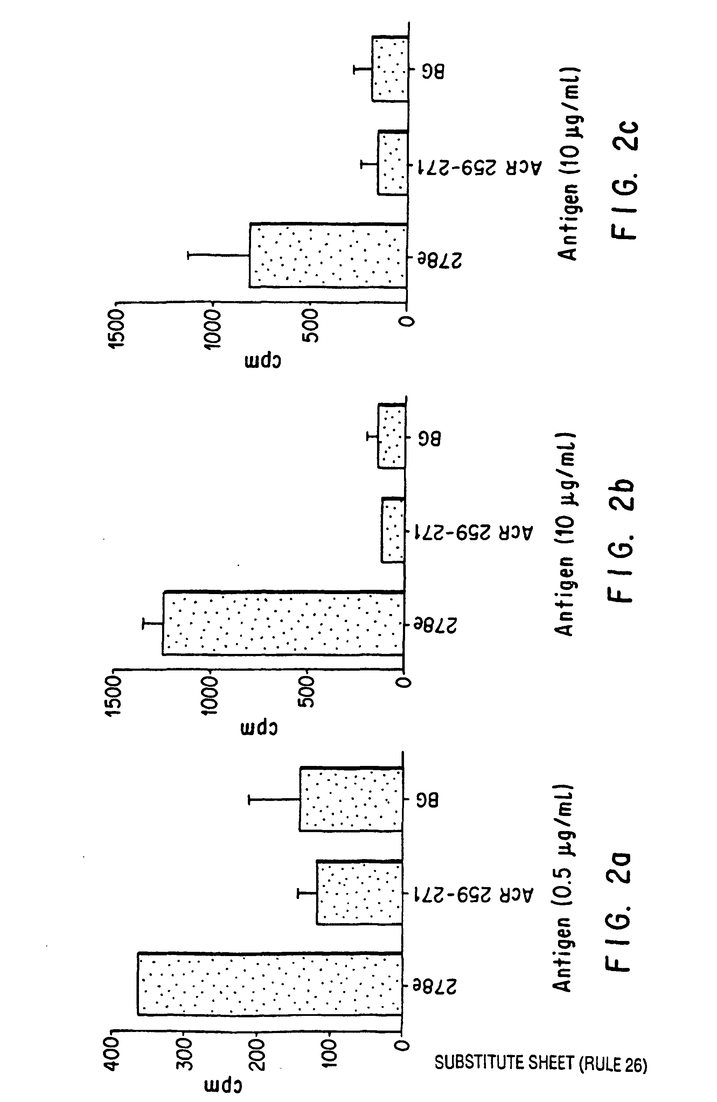

Fig. 2a-c demonstrates that peptide 278e was also recognized in the three different congenic B10 mouse strains. LNCs of B10.RIII mice (H-2^{r}) (Fig. 2a), B10.BR mice (H-2^{k}) (Fig. 2b) and B10.S mice (H-2') (Fig. 2c) showed significant higher proliferative responses to peptide 278e in the designated peptide concentrations than to the control peptide AcR259-271.

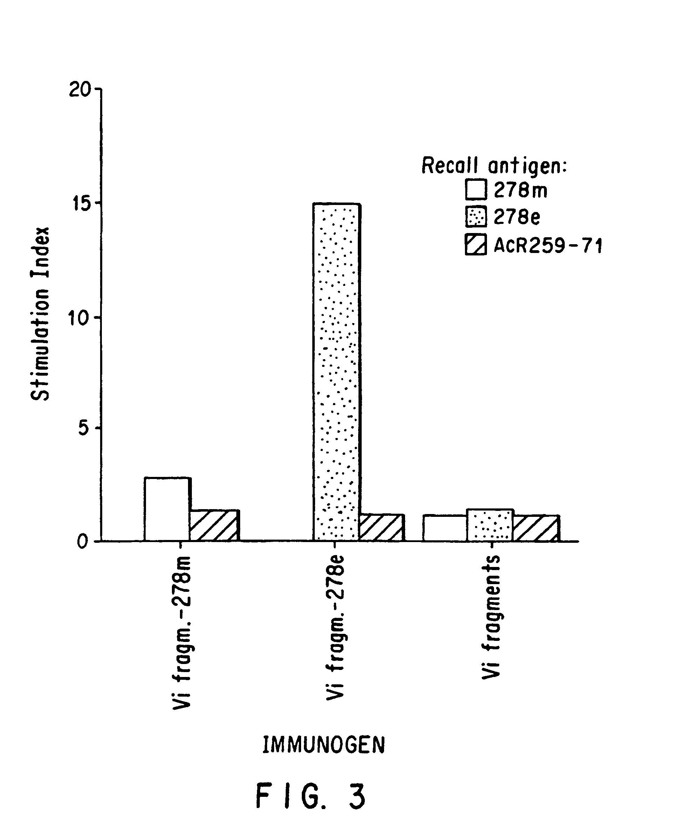

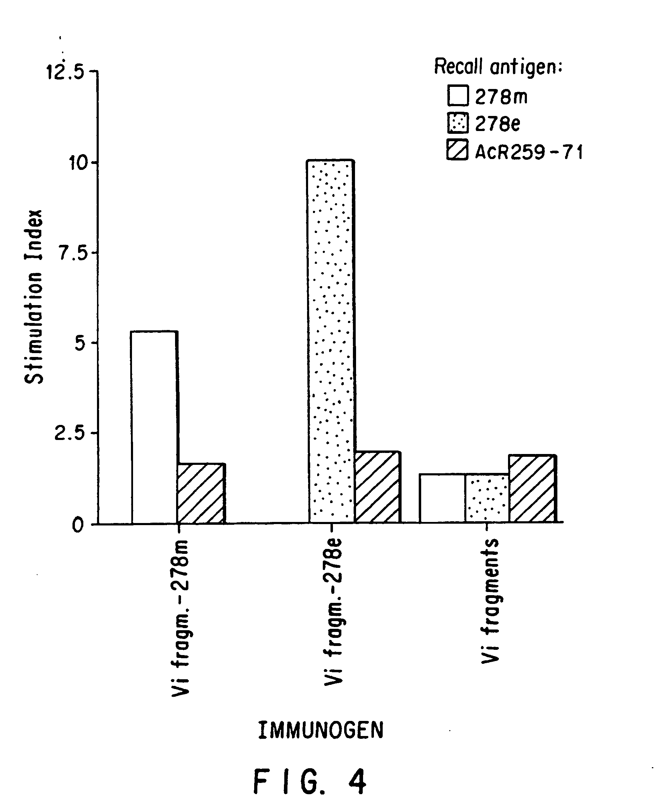

2.2. Lymph node cell proliferation to peptide 278e after immunization with peptide 278e conjugated to Vi-fragments. To analyze if coupling of peptide 278e to the polysaccharide Vi-fragments changes its antigenic structure, the LNC response to the peptide alone was tested after immunization with the sugar-peptide conjugate. Fig. 3 and Fig. 4 distinctly show that LNCs elicited by Vi-fragments-278e in BALB/c mice can recognize the unconjugated peptide when immunized with 2 µg Vi-fragments/mouse (Fig. 3) or 20 µg Vi-fragments/mouse (Fig. 4) as sugar-peptide conjugate (for the belonging injected peptide amount see Table 1).

**Table 1**

| Vi-fragment-peptide conjugate | Peptide amount injected per 2 µg Vi-fragment [µg] |
|---|---|
| Vi-fragments-278e | 0.8 |
| Vi-fragments-278m | 1.8 |
| Vi-fragments-278h | 2.2 |

### EXAMPLE 3. Antigenicity of Vi-fragments conjugated to peptide 278e.

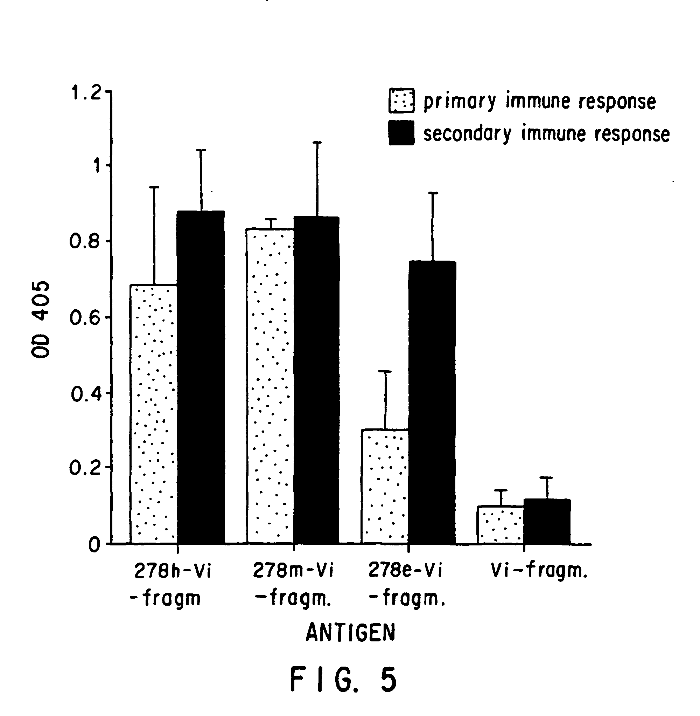

To examine if peptide 278e conjugated to Vi-fragments can enhance the immune response to this T-ind antigen, the immune response to the sugar was studied after inoculation of five BALB/c mice with the sugar-peptide conjugate. Fig. 5 clearly demonstrates that peptide 278e covalently linked to Vi-fragments can enhance the sugar-specific IgG antibody production substantially. Immunizing mice with a second dose of the conjugate gave rise to a strong booster effect indicating the involvement of T cells in the sugar-specific immune response. Inoculating BALB/c mice with the unconjugated polysaccharide only induced negligible levels of specific antibodies. The immune response induced by Vi-fragments-278e is compared to that elicited by the sugar conjugated to peptide 278h and 278m.

The above experiments offer evidence that peptide 278e can be recognized in association with a wide range of alleles of murine MHC molecules and can be used as carrier epitope for inducing enhanced immune responses to poorly immunogenic molecules. This evidence may be summarized a follows:
(i) Primed LNCs of mouse strains with varying genetic MHC-background were able to recognize peptide 278e by exhibiting specific proliferative responses.
(ii) Conjugating peptide 278e to Vi-fragments did not change its antigenic structure since LNCs primed with 278e coupled to the polysaccharide were still able to recognize the unbound peptide in an in vitro lymph node proliferation assay.
(iii) The immunogenicity of the Vi-fragments was increased when presented to the immune system as a conjugate coupled to peptide 278e.

The fact that LNCs that were primed with peptide 278e were not cross-reacting with the mouse homologue peptide 278m, indicates that peptide 278e used as carrier epitope probably will not induce immune responses directed to self components.

Since the immune response to peptide 278e seems not to be genetically restricted in mice, this synthetic peptide and analogs thereof might be used as universal carriers for the preparation of immunogenic conjugates to provide protective immunity against different pathogenic agents and can be suitable for the development of synthetic vaccines.

### REFERENCES

- Avery, O.T. and Goebel, W.F., J. Exp- Med. 50:533-550 (1929)
- Babbitt, B. et al., Nature, 317:359-361 (1985)
- Barrios, C., et al., Eur. J. Immunol. 22:1365-1372 (1992)
- Brett, S., J., Cease, K., B., & Berzofsky, J. A., J. Exp. Med., 168:357-373 (1988)
- Herzenberg, L. A. & Tokuhisa T., J. Exp. Med., 155: 1730-1740 (1982)
- Kappler, J., Roehn N., & P. Marrack, Cell, 49, -(1987)
- Kent, S. B. H., Hood, L. E., Beilan, H., Maister, S., & T. Geiser, Peptides by U. Ragnarsson, Stockholm (1984)
- Lussow, A. R. et al., Immunol. Letters 25:255-263 (1990)
- Lussow, A. R. et al., Eur. J. Immunol. 21:2297-2302 (1991)
- Munk, M.E. et al., Eur. J. Immunol. 18:1835-1838 (1988)
- Perraut, R., Lussow, A.R., Gavoille, S., Garraud, O., Matile, H., Tougne, C., van Embden, J., van der Zee, R. Lambert, P.-H., Gysin, J., & G. Del Giudice, Clin. Exp. Immunol., 93:382-386 (1993)
- Stone, A. L., & Szu S.C., J. Clin. Microbio., 26:719-725 (1988)
- Young, D. et.al., Proc. Natl. Acad. Sci. USA 85:4267-4270 (1988)

## Claims

1. A conjugate of a poorly immunogenic antigen and a synthetic peptide carrier constituting a T cell epitope derived from the sequence of E. *coli* hsp65 (GROEL) herein designated Pep278e which corresponds to positions 437-453 of the E. coli hsp65 molecule or an analog thereof wherein at least 70% of the electric properties and of the hydrophobicity of Pep278e are conserved, said peptide or analog thereof being capable of increasing substantially the immunogenicity of the poorly immunogenic antigen.

2. A conjugate according to claim 1 wherein the synthetic peptide carrier or analog is covalently bound to the poorly immunogenic antigen.

3. A conjugate according to claim 1 or 2 wherein the poorly immunogenic antigen is a peptide, a protein or a polysaccharide.

4. A conjugate according to claim 3 wherein the poorly immunogenic antigen is a peptide derived from HIV virus or from malaria antigen.

5. A conjugate according to claim 3 wherein the poorly immunogenic antigen is a bacterial polysaccharide.

6. A conjugate according to claim 1 wherein the synthetic peptide carrier, herein designated Pep278e, or analog of Pep278e correspond to positions 437-453 of the *E. coli* hsp65 molecule, having the sequence: 
437                                                    453
N E D Q N V G I K V A L R A M E A
 in which the residue N⁴³⁷ is either N or Q, the residue E⁴³⁸ is either E or D; the residue D⁴³⁹ is either D or E; the residue E⁴⁵² is either E or D; the residue Q⁴⁴⁰ is either Q or N, the residue K⁴⁴⁵ is either K or R; the residue V⁴⁴² is I, L, V, M, F, norleucine (Nle) or norvaline (Nva); the residue I⁴⁴⁴ is I, L, V, M, F, Nle or Nva; the residue V⁴⁴⁶ is, I, L, V, M, F, Nle or Nva; the residue L⁴⁴⁸ is L, I, V, M, F, Nle or Nva; the residue R⁴⁴⁹ is either R or K; and the residue M⁴⁵¹ is M, I, V, L, F, Nle or Nva.

7. A conjugate according to claim 1 wherein the synthetic peptide carrier or analog is directly bound to the poorly immunogenic antigen molecule.

8. A conjugate according to claim 7 wherein the poorly immunogenic antigen molecule is a bacterial polysaccharide.

9. A conjugate according to claim 8 wherein the bacterial polysaccharide is the capsular polysaccharide (CPS) Vi of Salmonella typhi.

10. A conjugate according to claim 1 wherein the synthetic peptide carrier or analog is linked to the poorly immunogenic antigen molecule through a spacer, selected from -O-R-CO-, NH-R-CO-, NH-R-NH, O-R-NH- or -NH-R-CH₂-, in which R is a saturated or unsaturated hydrocarbon chain.

11. A conjugate according to claim 10 wherein R is a saturated or unsaturated hydrocarbon chain substituted and/or interrupted by one or more aromatic radicals.

12. A conjugate according to claim 10 wherein R is a saturated or unsaturated hydrocarbon chain substituted and/or interrupted by one or more heteroatoms selected from N, O or S.

13. A conjugate according to claims 10-12 wherein R is an aliphatic hydrocarbon chain containing 3-16 carbon atoms.

14. A conjugate according to claim 13 wherein R is the residue of ε-aminocaproic acid.

15. A conjugate according to claim 14 of the formula 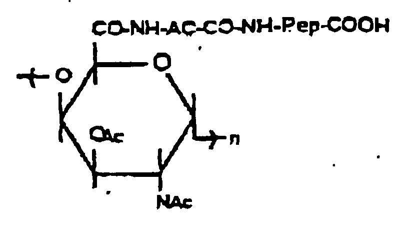 in which Ac is acetyl, AC is the residue of ε-aminocaproic acid, Pep is the residue of the peptide carrier Pep278e and the saccharide residue represents a repeating unit of the Vi capsular polysaccharide of Salmonella typhi.

16. A conjugate according to any of claims 1 to 15 able to produce a T lymphocyte helper effect resulting in an immune response characteristic for T-dependent antigens.

17. A conjugate according to any of claims 1 to 15 which on reinjection elicits a booster response resulting in increase in the level of antibodies to the poorly immunogenic antigen molecule.

18. A conjugate according to claim 17 which induces antibodies mainly of the IgG isotype.

19. A vaccine comprising a conjugate as claimed in claim 1.

20. A method for enhancing the immunogenicity of a poorly immunogenic antigen molecule which comprises linking it to a synthetic peptide carrier constituting a T cell epitope derived from the sequence of *E. coli* hsp65 herein designated Pep278e, which corresponds to positions 437-453 of the E. coli hsp65 molecule or an analog thereof, wherein at least 70% of the electric properties and of the hydrophobicity of Pep278e are conserved, said peptide or analog being capable of increasing substantially the immunogenicity of the poorly immunogenic antigen molecule.

21. A method of enhancing the immunogenicity of a poorly immunogenic antigen molecule which comprises mixing it to a synthetic peptide carrier constituting a T cell epitope derived from the sequence of E. coli hsp65 herein designated Pep278e, which corresponds to positions 437-453 of the E. coli hsp65 molecule or an analog thereof, wherein at least 70% of the electric properties and of the hydrophobicity of Pep278e are conserved, said peptide or analog being able to increase substantially the immunogenicity of the poorly immunogenic antigen molecule.

22. A method according to claim 20 to 21 for enhancing the immunogenicity of bacterial polysaccharides.

23. Use of a conjugate according to any preceding claim for the preparation of a vaccine for the immunization of a mammalian host.

24. Use of a synthetic peptide carrier constituting a T cell epitope derived from the sequence of *E. coli* hsp65 herein designated Pep278e, which corresponds to positions 437-453 of the E. coli hsp65 molecule or an analog thereof, in the manufacture of a medicament comprising said peptide or analog and a poorly immunogenic antigen molecule in the immunisation of a mammalian host, wherein at least 70% of the electric properties and of the hydrophobicity of Pep278e are conserved, said peptide or analog being capable of enhancing substantially the immunogenicity of the poorly immunogenic antigen.

25. Use of a synthetic peptide carrier according to claim 6 in the manufacture of a medicament comprising said peptide or analog and a poorly immunogenic antigen molecule in the immunization of a mammalian host, wherein said peptide or analog is able to enhance substantially the immunogenicity of the poorly immunogenic antigen.

## Patentansprüche

1. Konjugat eines schwach immunogenen Antigens und eines synthetischen Peptidträgers, welcher ein T-Zellepitop darstellt, das von der Sequenz von E. coli hsp65 (GROEL), die hierin Pep278e genannt wird, welche den Positionen 437-453 des E. coli hsp65-Moleküls entspricht, abgeleitet ist, oder ein Analog desselben, wobei zumindest 70 % der elektrischen Eigenschaften und der Hydrophobizität von Pep278e bewahrt bleiben, wobei das Peptid oder das Analog desselben in der Lage ist, die Immunogenizität des schwach immunogenen Antigens maßgeblich zu vergrößern.

2. Konjugat gemäß Anspruch 1, wobei der synthetische Peptidträger oder das Analog kovalent am schwach immunogenen Antigen gebunden ist.

3. Konjugat gemäß Anspruch 1 oder 2, wobei das schwach immunogene Antigen ein Peptid, ein Protein oder ein Polysaccharid ist.

4. Konjugat gemäß Anspruch 3, wobei das schwach immunogene Antigen ein aus HIV-Virus oder aus Malaria-Antigen erhaltenes Peptid ist.

5. Konjugat gemäß Anspruch 3, wobei das schwach immunogene Antigen ein bakterielles Polysaccharid ist.

6. Konjugat gemäß Anspruch 1, wobei der synthetische Peptidträger, der hier als Pep278e bezeichnet wird, oder ein Analog von Pep278e den Positionen 437-453 des E. coli hsp65-Moleküls entspricht, welches die Sequenz aufweist: 
4 3 7                                    4 5 3
N E D Q N V G I K V A L R A M E A
 in dem der Rest N⁴³⁷ entweder N oder Q ist; der Rest E⁴³⁸ entweder E oder D ist; der Rest D⁴³⁹ entweder D oder E ist; der Rest E⁴⁵² entweder E oder D ist; der Rest Q⁴⁴⁰ entweder Q oder N ist; der Rest K⁴⁴⁵ entweder K oder R ist; der Rest V⁴⁴² I, L, V, M, F, Norleucin (Nle) oder Norvalin (Nva) ist; der Rest I⁴⁴⁴ I, L, V, M, F, Nle oder Nva ist; der Rest V⁴⁴⁶ I, L, V, M, F, Nle oder Nva ist; der Rest L⁴⁴⁸ L, I, V, M, F, Nle oder Nva ist; der Rest R⁴⁴⁹ entweder R oder K ist; und der Rest M⁴⁵¹ M, I, V, L, F, Nle oder Nva ist.

7. Konjugat gemäß Anspruch 1, wobei der synthetische Peptidträger oder sein Analog direkt an das schwach immogene Antigenmolekül gebunden ist.

8. Konjugat gemäß Anspruch 7, wobei das schwach immunogene Antigenmolekül ein bakterielles Polysaccharid ist.

9. Konjugat gemäß Anspruch 8, wobei das bakterielle Polysaccharid das Kapsel-Polysaccharid (CPS) Vi aus Salmonella typhi ist.

10. Konjugat gemäß Anspruch 1, wobei der synthetische Peptidträger oder sein Analog mit dem schwach immunogenen Antigenmolekül durch einen Abstandhalter verknüpft ist, der ausgewählt ist aus -O-R-CO-, NH-R-CO-, NH-R-NH, O-R-NH- oder -NH-R-CH₂-, wobei R eine gesättigte oder ungesättigte Kohlenwasserstoffkette ist.

11. Konjugat gemäß Anspruch 10, wobei R eine gesättigte oder ungesättigte Kohlenwasserstoffkette ist, die durch eine oder mehrere aromatische Reste substituiert und/oder unterbrochen ist.

12. Konjugat gemäß Anspruch 10, wobei R eine gesättigte oder ungesättigte Kohlenwasserstoffkette ist, die durch ein oder mehrere aus N, O oder S ausgewählte Heteroatome substituiert und/oder unterbrochen ist.

13. Konjugat gemäß den Ansprüchen 10 bis 12, wobei R eine aliphatische Kohlenwasserstoffkette ist, die 3-16 Kohlenstoffatome enthält.

14. Konjugat gemäß Anspruch 13, wobei R der Rest der ε-Aminocapronsäure ist.

15. Konjugat gemäß Anspruch 14 der Formel 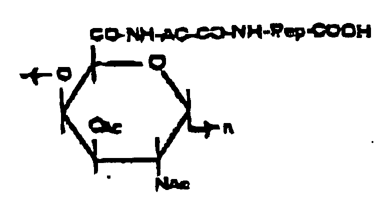 wobei Ac Acetyl ist, AC der Rest der ε-Aminocapronsäure ist, Pep der Rest des Peptidträgers Pep278e ist und der Saccharidrest eine Wiederholungseinheit des Vi-Kapselpolysaccharids von Salmonella typhi repräsentiert.

16. Konjugat gemäß einem der Ansprüche 1 bis 15, das in der Lage ist, einen T Lymphozytenhelfereffekt zu erzeugen, der zu einer für T-abhängige Antigene charakteristischen Immunantwort führt.

17. Konjugat gemäß einem der Ansprüche 1 bis 15, das bei Reinjektion eine Boosterreaktion hervorruft, die zu einem Anstieg der Menge von Antikörpern gegen das schwach immunogene Antigenmolekül führt.

18. Konjugat gemäß Anspruch 17, welches Antikörper hauptsächlich des IgG-Isotyps induziert.

19. Vakzin, umfassend ein Konjugat, wie in Anspruch 1 beansprucht.

20. Verfahren zum Verstärken der Immunogenizität eines schwach immunogenen Antigenmoleküls, welches sein Verbinden mit einem synthetischen Peptidträger umfasst, der ein T-Zellepitop darstellt, das von der Sequenz von E. coli hsp65, hier als Pep278e bezeichnet, welches den Positionen 437-453 des E. coli hsp65-Moleküls entspricht, abgeleitet ist, oder ein Analog desselben, wobei zumindest 70 % der elektrischen Eigenschaften und der Hydrophobizität von Pep278e bewahrt sind, wobei das Peptid oder Analog in der Lage ist, die Immunogenizität des schwach immunogenen Antigenmoleküls maßgeblich zu vergrößern.

21. Verfahren zum Verstärken der Immunogenizität eines schwach immunogenen Antigenmoleküls, welches sein Mischen mit einem synthetischen Peptidträger umfasst, der ein T-Zellepitop darstellt, das von der Sequenz von E. coli hsp65, hier als Pep278e bezeichnet, welches den Positionen 437-453 des E. coli hsp65-Moleküls entspricht, abgeleitet ist, oder ein Analog desselben, wobei zumindest 70 % der elektrischen Eigenschaften und der Hydrophobizität von Pep278e bewahrt sind, wobei das Peptid oder Analog in der Lage ist, die Immunogenizität des schwach immunogenen Antigenmoleküls maßgeblich zu vergrößern.

22. Verfahren gemäß Anspruch 20 oder 21 zum Verstärken der Immunogenizität von bakteriellen Polysacchariden.

23. Verwendung eines Konjugats gemäß einem der vorstehenden Ansprüche für die Herstellung eines Vakzins zur Immunisierung eines Säugerwirts.

24. Verwendung eines synthetischen Peptidträgers, der ein T-Zellepitop darstellt, das von der Sequenz von E. coli hsp65, die hier als Pep278e bezeichnet wird, welche den Positionen 437-453 des E. coli hsp65-Moleküls entspricht, abgeleitet ist, oder ein Analog desselben, bei der Herstellung eines Medikamentes, welches das Peptid oder Analog und ein schwach immunogenes Antigenmolekül bei der Immunisierung eines Säugerwirts umfasst, wobei zumindest 70 % der elektrischen Eigenschaften und der Hydrophobizität von Pep278e bewahrt sind, wobei das Peptid oder Analog in der Lage ist, die Immunogenizität des schwach immunogenen Antigens maßgeblich zu verstärken.

25. Verwendung eines synthetischen Peptidträgers gemäß Anspruch 6 in der Herstellung eines Medikaments, welches das Peptid oder Analog und ein schwach immunogenes Antigenmolekül bei der Immunisierung eines Säugerwirts umfasst, wobei das peptid oder Analog in der Lage ist, die Immunogenizität des schwach immunogenen Antigens maßgeblich zu verstärken.

## Revendications

1. Conjugué d'un antigène faiblement immunogène et d'un porteur peptidique synthétique constituant un épitope de lymphocyte T dérivé de la séquence de *E. coli* hsp65 (GROEL) nommé ici Pep278e, qui correspond aux positions 437-453 de la molécule hsp65 de *E. coli*, ou un analogue de celui-ci dans lequel au moins 70 % des propriétés électriques et de l'hydrophobicité de Pep278e sont conservées, ledit peptide ou analogue de celui-ci étant capable d'accroître sensiblement l'immunogénicité de l'antigène faiblement immunogène.

2. Conjugué selon la revendication 1 dans lequel le porteur peptidigue synthétique ou son analogue sont liés de manière covalente à l'antigène faiblement immunogène.

3. Conjugué selon la revendication 1 ou 2 dans lequel l'antigène faiblement immunogène est un peptide, une protéine ou un polysaccharide.

4. Conjugué selon la revendication 3 dans lequel l'antigène faiblement immunogène est un peptide dérivé du virus VIH ou de l'antigène du paludisme.

5. Conjugué selon la revendication 3 dans lequel l'antigène faiblement immunogène est un polysaccharide bactérien.

6. Conjugué selon la revendication 1 dans lequel le porteur peptidique synthétique, ici nommé Pep278e, ou l'analogue de Pep278e, correspondent aux positions 437-453 de la molécule hsp65 de *E. coli*, ayant la séquence : 
437 453
N E D Q N V G I K V A L R A M E A
 dans laquelle le résidu N⁴³⁷ est N ou Q, le résidu E⁴³⁸ est E ou D ; le résidu D⁴³⁹ est D ou E ; le résidu E⁴⁵² est E ou D ; le résidu Q⁴⁴⁰ est Q ou N ; le résidu K⁴⁴⁵ est K ou R ; le résidu V⁴⁴² est I, L, V, M, F, norleucine (Nle) ou norvaline (Nva) ; le résidu I⁴⁴⁴ est I, L, V, M, F, Nle ou Nva ; le résidu V⁴⁴⁶ est I, L, V, M, F, Nle ou Nva ; le résidu L⁴⁴⁸ est L, I, V, M, F, Nle ou Nva ; le résidu R⁴⁴⁹ est R ou K ; et le résidu M⁴⁵¹ est M, I, V, L, F, Nle ou Nva.

7. Conjugué selon la revendication 1 dans lequel le porteur peptidique synthétique ou son analogue sont directement liés à la molécule antigénique faiblement immunogène.

8. Conjugué selon la revendication 7, dans lequel la molécule antigénique faiblement immunogène est un polysaccharide bactérien.

9. Conjugué selon la revendication 8 dans lequel le polysaccharide bactérien est le polysaccharide capsulaire (CPS) Vi de *Salmonella typhi*.

10. Conjugué selon la revendication 1 dans lequel le porteur peptidique synthétique ou son analogue sont liée à la molécule antigénique faiblement immunogène par un espaceur, choisi parmi -O-R-CO-, NH-R-CO-, NH-R-NH, O-R-NH- ou -NH-R-CH₂-, dans lequel R est une chaîne d'hydrocarbure saturée ou non saturée.

11. Conjugué selon la revendication 10 dans lequel R est une chaîne d'hydrocarbure saturée ou non saturée substituée et/ou interrompue par un ou plusieurs radicaux aromatiques.

12. Conjugué selon la revendication 10 dans lequel R est une chaîne d'hydrocarbure saturée ou non saturée substituée et/ou interrompue par un ou plusieurs hétéroatomes choisis parmi N, O ou S.

13. Conjugué selon les revendications 10 à 12 dans lequel R est une chaîne d'hydrocarbure aliphatique contenant 3 à 16 atomes de carbone.

14. Conjugué selon la revendication 13 dans lequel R est le résidu d'acide ε-aminocaproïque.

15. Conjugué selon la revendication 14 de formule 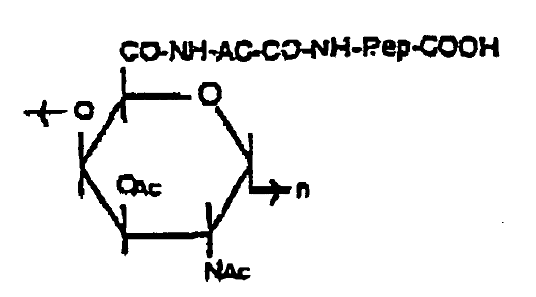 dans laquelle Ac est acétyle, AC est le résidu d'acide ε-aminocaproïque, Pep est le résidu du porteur peptidique Pep278e et le résidu saccharide représente un motif répété du polysaccharide capsulaire Vi de *Salmonella typhi*.

16. Conjugué selon l'une quelconque des revendications 1 à 15 capable de produire un effet de lymphocyte T auxiliaire aboutissant à une réponse immunitaire caractéristique des antigènes T-dépendants.

17. Conjugué selon l'une quelconque des revendications 1 à 15 qui suite à une réinjection provoque une réponse plus forte aboutissant à une augmentation du niveau d'anticorps dirigés contre la molécule antigénique faiblement immunogène.

18. Conjugué selon la revendication 17 qui induit des anticorps principalement de l'isotype IgG.

19. Vaccin comprenant un conjugué selon la revendication 1.

20. Procédé pour augmenter l'immunogénicité d'une molécule antigénique faiblement immunogène qui comprend l'étape consistant à lier cette dernière à un porteur peptidique synthétique constituant un épitope de lymphocyte T dérivé de la séquence de *E. coli* hsp65, ici nommée Pep278e, qui correspond aux positions 437-453 de la molécule hsp65 de *E*. *coli*, ou un analogue de celui-ci, dans lequel au moins 70 % des propriétés électriques et de l'hydrophobicité de Pep278e sont conservées, ledit peptide ou analogue étant capables d'accroître sensiblement l'immunogénicité de la molécule antigénique faiblement immunogène.

21. Procédé pour augmenter l'immunogénicité d'une molécule antigénique faiblement immunogène qui comprend l'étape consistant à mélanger cette dernière avec un porteur peptidique synthétique constituant un épitope de lymphocyte T dérivé de la séquence de *E. coli* hsp65, ici nommée Pep278e, qui correspond aux positions 437-453 de la molécule hsp65 de *E.* coli, ou un analogue de celui-ci, dans lequel au moins 70 % des propriétés électriques et de l'hydrophobicité de Pep278e sont conservées, ledit peptide ou analogue étant capables d'accroître sensiblement l'immunogénicité de la molécule antigénique faiblement immunogène.

22. Procédé selon les revendications 20 et 21 pour augmenter l'immunogénicité de polysaccharides bactériens.

23. Utilisation d'un conjugué selon l'une quelconque des revendications précédentes pour préparer un vaccin pour l'immunisation d'un hôte mammifère.

24. Utilisation d'un porteur peptidique synthétique constituant un épitope de lymphocyte T dérivé de la séquence de *E. coli* hsp65, ici nommée Pep278e, qui correspond aux positions 437-453 de la molécule hsp65 de *E. coli*, ou un analogue de celui-ci, pour fabriquer un médicament comprenant ledit peptide ou analogue et une molécule antigénique faiblement immunogène pour l'immunisation d'un hôte mammifère, dans lequel au moins 70 % des propriétés électriques et de l'hydrophobicité de Pep278e sont conservées, ledit peptide ou analogue étant capable d'améliorer sensiblement l'immunogénicité de la molécule antigénique faiblement immunogène.

25. Utilisation d'un porteur peptidique synthétique selon la revendication 6 pour fabriquer un médicament comprenant ledit peptide ou analogue et une molécule antigénique faiblement immunogène pour l'immunisation d'un hôte mammifère, dans lequel ledit peptide ou analogue sont capables d'améliorer sensiblement l'immunogénicité de l'antigène faiblement immunogène.
